# EUROPÄISCHE PATENTANMELDUNG

(11) **EP 0 909 766 A2**
(43) Veröffentlichungstag der Anmeldung: **21.04.1999**
(21) Anmeldenummer: 98118705.7
(22) Anmeldetag: 02.10.1998
(51) Int. Cl.: C08B 11/20, C08B 15/06, C08B 15/10, C09D 7/12, C09D 11/14, D06P 1/50, A61K 7/00, A61K 47/38, C09J 11/08

(54) **Urethan-modifizierte nichtionische Cellulosen, ein Verfahren zu deren Herstellung und deren Verwendung als verdickend wirkendes Zusatzmittel**

(30) Priorität: 15.10.1997 DE 19745463; 07.04.1998 DE 19815635
(71) Anmelder: Borchers GmbH, 40789 Monheim (DE)
(72) Erfinder: Günter, Link, 38644 Goslar (DE); Edelmann, Dirk Dr., 42277 Wuppertal (DE)
(74) Vertreter: Steiling, Lothar, Dr.

(57) **Zusammenfassung**

Cellulosen und hydroxy-funktionalisierte Cellulosen werden mit poIyfunktionellen Isocyanaten so modifiziert, daß Cellulosederivate gebildet werden, die eine verlängerte Anquellzeit in Wasser besitzen. Bevorzugt werden dabei Hydroxyethyl(propyl)cellulosen und niedrigveretherte Carboxymethylcellulosen umgesetzt, deren erfindungsgemäße Reaktionsprodukte eingesetzt als Verdickungsmittel besonders günstige rheologische Eigenschaften besitzen.

## Beschreibung

Die Erfindung betrifft neue Klassen von modifizierten nicht-ionischen wasserlöslichen Polymeren. Speziell bezieht sie sich auf modifizierte nicht-ionische wasserlösliche Cellulosen.

Nichtionische wasserlösliche Cellulosen werden in einem weiten Feld von industriellen Anwendungsgebieten eingesetzt, so z.B. als Verdicker, als Wasserrückhaltesubstanzen, als Dispergierhilfsmittel, in Polymerisationsprozessen, in Klebstoffen usw.. Für einige dieser Anwendungen werden spezielle Cellulosetypen benötigt, für andere kann ein breites Spektrum von Cellulosen eingesetzt werden.

Die häufigsten modifizierten Cellulosen gehören zur Klasse der nicht-ionischen Celluloseether und Celluloseester. Dazu gehören z.B. Methylcellulosen, Hydroxypropylmethylcellulosen, Hydroxyethylcellulosen, Celluloseacetobutyrate usw.

Besonders wichtig für die vorliegende Erfindung ist die Betrachtung der Verdickungswirkung der o.g. Cellulosetypen in Wasser bzw. wasserverdünnbaren Bindemitteln und Farben. Dabei gilt generell, wie für alle hochmolekularen Polymere, daß die Verdickungswirkung in Wasser mit dem Molekulargewicht ansteigt. Die Herstellung hochmolekularer Cellulosen wird aber durch die Natur der Ausgangsstoffe, z.B. den Baumwoll-Linters und anderen Zellstoffen, begrenzt. Die nun für die Gewinnung wasserquellbarer und damit verdickender Cellulosen notwendige Hydrophobierung vor allem über Veretherung führt aber in den häufigsten Fällen auch zu Molekulargewichtserniedrigungen durch Nebenreaktionen. Außerdem sind hochmolekulare Cellulosen sehr viel schwieriger zu verarbeiten, so können polymeranaloge Reaktionen an solchen Systemen aufgrund der inhomogenen Verteilung der hochmolekularen Cellulosegrundkörper nur unzureichend durchgeführt werden.

Es ist deshalb Stand der Technik, niedrig-molekulare Cellulosen zu erzeugen, die durch polymeranaloge Reaktionen wie z.B. Veretherung mit Epoxiden oder Chloralkyl-verbindungen zu modifizierten Cellulosen umgesetzt werden, welche hochviskose Lösungen in Wasser ergeben. Derartig modifizierte Celluloseether (und auch Ester) weisen als Stand der Technik eine hohe Oberflächenaktivität auf und können zur Herstellung viskoser wässriger Lösungen herangezogen werden. Die Darstellung solcher Ether ist schon seit 1939 bekannt (US 2160783).

Das Hauptprodukt derartiger Umsetzungen sind Hydroxyethyl- bzw. Hydroxypropylcellulosen, die aber aufgrund ihres stark hydrophilen Charakters eine kleine Wasserlöslichkeit besitzen und deshalb nicht ein optimales, d.h. ein nicht maximales Quellverhalten ergeben. Weiterhin lassen sich derartige Cellulosen technisch nur schwierig zu hochviskosen Lösungen in Wasser verarbeiten, da sie ein stark inhomogenes weil zu schnelles Löse- und Anquellvermögen in Wasser zeigen.

Es ist daher Stand der Technik, solche Cellulosen zu 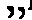 hydrophobieren", mit dem Ziel die Anquellzeit bzw. die Lösezeit der Cellulosederivate zu verlängern. Die o.g. hydroxyfunktionellen Cellulosen werden dabei nach dem Stand der Technik sowohl mit kurzkettigen Alkylgruppen (z.B. Methyl-,Ethyl-, Propyl- oder Butylrest) versetzt als auch mit längerkettigen Alkylgruppen (C10-C24) umgesetzt. Diese Techniken sind z.B. beschrieben in US 3091542, US 3272640, US 3435027 oder US 4228277. Dabei sind die Umsetzungsraten (Substitutionsgrade) verschieden hoch und es resultieren je nach Herstellungsverfahren Cellulosen mit ganz unterschiedlichen Eigenschaften, die auch von dem unterschiedlichen Verzweigungsgrad und Polymerisationsgrad der Celluloseketten herrühren. Eine Übersicht der als Stand der Technik benutzten Cellulosen zum Verdicken von wasserverdünnbaren Lacksystemen findet sich z.B. in Surf. Int. Coating (1995) 78, S. 285-288.

Alle für diesen Zweck eingesetzten nicht-ionischen wasserlöslichen Cellulosen weisen dabei als gemeinsames Merkmal auf daß die Hydrophierungsgruppen über Veretherungsreaktionen oder Veresthetungsreaktionen an die hydroxyfunktionellen Celluloseether angebunden wurden.

Zusätzlich gibt es auch modifizierte Celullosen, die zusätzlich Carboxygruppen enthalten. Aber auch diese werden nach dem Stand der Technik über Veretherungs- bzw. Veresterungsreaktionen modifiziert. Solche Carboxycellulosen können auch mit Basen umgesetzt werden z.B. NaOH oder Aminen. Dabei erhält man im Gegensatz zu erfindungsgemäßen Umsetzungen ionische Cellulosen, deren Wirkung in Wasser pH-Wert abhängig ist.

Weiterhin sind Umsetzungen an Celluloseethern nicht literaturbekannt, bei denen über die o.g. Reaktionen Celluloseketten zumindest anteilig vernetzt werden.

Gegenstand der vorliegenden Erfindung ist es, modifizierte nicht-ionische wasserlösliche Cellulosen bereitzustellen, die nicht über Veretherungen oder Veresterungen gewonnen werden. Außerdem sollten gleichfalls Cellulosen bereitgestellt werden, die anteilig vorvernetzt sind. Diese Vorvernetzung hätte den Vorteil, daß die mit solchen Cellulosen verdickten wässrigen Lösungen, Bindemittel oder Lacke gegenüber der Einwirkung von Scherkräften stabiler wären als solche o.g. Systeme, die eine solche Vorvernetzung nicht aufweisen. Diese Cellulosen sollen nach ihrer Modifizierung aber weiterhin wasserlöslich sein und hochviskose Lösungen in Wasser ergeben und somit als Verdickungsmittel für wässrige Zubereitungen wirksam sein.

Diese Aufgabe konnte überraschend gelöst werden durch die Umsetzung von hydroxyfunktionellen Celluloseethern mit polyfunktionellen Isocyanaten.

Im Sinne der vorliegenden Erfindung können dabei alle wasserlöslichen Cellulosen und Celluloseether bzw. Ester mit freien OH-Gruppen als Ausgangsmaterialien dienen. Das sind z.B. Hydroxyethyl-, Hydroxypropyl-, Methyl- (DS< 2,5), Ethylhydroxyethyl- oder Methylhydroxyethylcellulosen (DS<2,0). Ebenfalls geeignet sind Carboxycellulosen wie Carboxymethyl- oder Carboxyethylcellulosen mit DS<2,0.

Die bevorzugten Cellulosetypen sind Hydroxyethyl- bzw. Hydroxypropylcellulosen mit einem mittleren Molekulargewicht von 50.000 bis 500.000 Dalton. Ebenfalls bevorzugt sind Carboxymethylcellulosen mit DS<1,5 mit mittleren Molekulargewichten von 20.000 bis 500.000 Dalton bzw. einem Polymerisationsgrad von ca. 70 bis 2.000 Einheiten.

Weitere bevorzugte Cellulosen sind wasserlösliche Hydroxyethyl- und Hydroxypropylcellulosen mit Molekulargewichten von 20.000 bis 500.000 Dalton, die viskose Lösungen in Wasser ergeben. Ebenso bevorzugt sind Carboxymethyl- und Carboxyethylcellulosen mit Molekulargewichten von 20.000 bis 500.000 Dalton, die viskose Lösungen in Wasser ergeben.

Die erfindungsgemäßen polyfunktionellen Isocynate können aliphatischer oder aromatischer Natur sein. Beispiele solcher erfindungsgemäßen Isocyanate sind Toluoldiisocyanat, Isophorondiisocyanat, Hexamethylendiisocyanat, Methylen-bis-(phenylisocyanat) oder Triisocyanatocyanurat. Besonders bevorzugt sind aliphatische Diisocyanate wie 4,4'-Methylen-bis-(cyclohexylisocyanat) oder 1,6-Hexamethylendiisocyanat.

Die Methoden für die Herstellung der erfindungsgemäßen Produkte sind analog den bekannten Methoden zur Umsetzung von organischen hydroxyfunktionellen Verbindungen mit Isocyanaten. Bervorzugt ist dabei die Methode, bei der die Cellulose bzw. eine andere o.g. erfindungsgemäße hydroxyfunktionelle Cellulose in einem geeigneten organischen Lösemittel aufgeschlämmt wird und zu diesem Ansatz dann das entsprechende Isocyanat gegeben wird. Nach Beendigung der Reaktion, gegebenenfalls unter Erwärmung und/oder Zuhilfenahme von Katalysatoren, wird die umgesetzte Cellulose abfiltriert. Als Lösemittel kommen dabei alle inerten organischen Lösemittel in Betracht, wie z.B. Ketone (Aceton, Methyisobutyketon), Aromaten (Toluol, Xylol) oder Methylenchlorid bzw. Chloroform oder Testbenzine bzw. entsprechende Mischungen aliphatischer Verbindungen ( 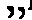 Isopar"-Stoffe). Das bevorzugte Lösemittel hängt dabei von der Alt der Cellulose und dem gewählten Isocyanat ab. Im Sinne der Erfindung sind dabei nur Umsetzungen, bei denen der Gewichtsanteil des eingesetzten polyfunktionellen Isocyanats nicht mehr als 10 Gew.% an dem der umzusetzenden Cellulose ist. Die bevorzugten erfindungsgemäßen Zugabemengen der polyfunktionellen Isocyanate liegen zwischen 0,2 und 5 Gew.% Isocyanat bezogen auf die umzusetzende Menge an Cellulose bzw. Cellulosether.

Ein weiterer Gegenstand der vorliegenden Erfindung ist die Verwendung solcher umgesetzten Cellulosen als rheologische Additive zur Verdickung von wässrigen bzw. überwiegend wässrigen Systemen wie z.B. Dispersionsfarben, Druck -und Pigmentpasten, Füllstoff- und Pigmentdispersionen, Papier-, Leder- und Textilhilfsmitteln, Zubereitungen für die Erdölförderung, Zubereitungen von Waschmitteln, Klebstoffen, Wachsen, Polituren, Formulierungen für pharmazeutische und veterinäre Zwecke, Pflanzenschutzzubereitungen, kosmetische Artikel, vorzugsweise Shampoos und Waschgele usw. Auch Wasser selbst kann mit den erfindinngsgemäßen Verdickern verdickt werden, um dann gegebenenfalls mit weiteren Zusätzen versetzt zu werden oder selbst zu wässrigen Zubereitungen zugesetzt zu werden.

Die erfindungsgemäß modifizierten Cellulosen bzw. deren Zubereitungen eignen sich nicht nur zur Verdickung von rein wässrigen Systemen, sondern auch von solchen Systemen, die anteilsmäßig organische Lösemittel oder andere flüchtige organische Zusatzmittel enthalten, wie z.B Alkohole, Glykole, Ketone usw. Alle erfindungsgemäß verdickbaren Systeme können die üblichen Hilfs- und Zusatzmittel wie z.B. Entschäumer, Fließ- und Verlaufsmittel, Netzmittel, Füllstoffe, Pigmente usw. enthalten.

Beispiele für erfindungsgemäß verdickbare Systeme sind wässrige Polyacrylatdispersionen, wässrige Lösungen bzw. Dispersionen von Mischpolymerisaten olefinsich ungesättigter Monomere, wässrige Polyvinylacetatdispersionen, wässrige Polyesterdispersionen sowie entsprechende Emulsionen der o.g. Bindemittel Weitere Beispiele sind insbesondere auch gebrauchsfertige Zubereitungen der oben beschriebenen Art auf Basis derartiger Dispersionen.

Der Vorteil bei der Verwendung der erfindinngsgemäßen modifizierten Cellulosen liegt in der Veränderung der rheologischen Profils solcher Systeme gegenüber demjenigen Profil, welches durch eine entsprechende nicht-erfindungsgemäße Cellulose erreicht wird. Insbesondere wird die Spritzneigung von mit den o.g. Cellulosen hergestellten Lacken und Farben deutlich verringert, die Löseverzögerung vergrößert und damit die Herstellung von wässrigen Anquellungen erleichtert. Das Ab- bzw. Verlaufverhalten sowie die Obenflächenbeschaffenheit der mit den erfindinngsgemäßen Cellulosen hergestellten Lackfilme wird verbessert. In einer Reihe von Fällen kann auch die Benetzung organischer Pigmente (z.B. in Abtönpasten) verbessert werden.

### Beispiele

### 1. Herstellung der erfindungsgemäßen Verbindungen

### 1.1. Modifizierungen an einer niedrig veretherten Methylhydroxyethylcellulose.

Zu 15,0 g einer niedrig veretherten Methylhydroxyethylcellulose (MW 20000 JB der Wolff Walsrode AG) wurden in einem 250 ml Dreihalsrundkolben (mit Rührer und Rückflußkühler) 25 ml Xylol (technisches Isomerengemisch) gegeben und durch Rühren aufgeschlämmt. Zu diesem Gemisch wurden dann bei Raumtemperatur 1,5 g einer 10 gew.%-igen Lösung von Desmodur® W (4,4'- Methylen-bis-(cyclohexylisocyanat) der Bayer AG) in Xylol gegeben sowie zusätzlich 0,15 g Octa-Soligen Zinn® 28 (der Borchers GmbH). Der Reaktionsansatz wurde anschließend 1h bei 50°C gerührt. Die so modifizierte Cellulose wird nun abfiltriert und bei Raumtemperatur 24 h getrocknet. (Cellulose **1A**).

In einem analogen Reaktionsverfahren wurden 15,0 g der niedrig veretherten Methylhydroxyethylcellulose mit 3,0 g einer 10 gew.%igen Lösung von Desmodur® W (s.o.) in Xylol und 0,15 g Octa-Soligen® Sn 28 (s.o.) versetzt und ebenfalls 1h bei 50°C gerührt. (Cellulose **1B**).

### 1.2. Modifizierungen an einer Hydroxyethylcellulose

Zu 15,0 g einer Hydroxyethylcellulose (Natrosol® 250 H4BR der Aqualon East BV) wurden in einem 250 ml Dreihalsrundkolben (mit Rührer und Rückflußkühler) 25 ml Xylol (technisches Isomerengemisch) gegeben und durch Rühren aufgeschlämmt. Zu diesem Gemisch wurden dann bei Raumtemperatur 1,5 g einer 10 gew.%igen Lösung von Desmodur W (s.o.) in Xylol (technisches Isomerengemisch) und 0,15 g Octa-Soligen® Sn 28 (s.o.) gegeben. Dieser Reaktionsansatz wurde anschließend 1h bei 50°C gerührt. Die so modifizierte Cellulose wird nun abfiltriert und bei Raumtemperatur 24 h getrocknet. (Cellulose **2A**).

In einem analogen Reaktionsverfahren wurden zu 15,0 g Natrosol® 250 H4BR 3,0 g einer 10 gew.%igen Lösung von Desmodur W (s.o.) in Xylol (technisches Isomerengemisch) und 0,15 g Octa-Soligen® Sn 28 (s.o.) gegeben und ebenfalls 1h bei 50°C gerührt. Die modifizierte Cellulose wurde abfiltriert und 24h bei Raumtemperatur getrocknet. (Cellulose **2B**).

### 2. Herstellung von wässrigen Lösungen Von den in Beispiel 1 genannten modifizierten Cellulosen wurden jeweils

2 gew.%ige Lösungen in Wasser angesetzt. Dazu wurden 390,0 g VE-Wasser, 1 ml Entschäumer T (Tri-n-butylphosphat der Firma Borchers GmbH), 8,0 g der entsprechenden Cellulose in einem 500 ml Schraubdeckelglas eingewogen, verrührt. Zu diesem Gemisch wurden anschließend 2,0 g konz. Ammoniakwasser gegeben und verrührt. Der gesamte Ansatz wurde dann 3h auf eine Flaschenrolle mit einer Umdrehungszahl von 1 s⁻¹ gelegt. Abschließend wird der Ansatz 24 h bei Raumtemperatur stehen gelassen.

Die Bestimmung der Löseverzögerung (Anquellungszeit) ergab folgende Werte: (Tab.1.)

**Tabelle 1**

| Ergebnisse der Löseverzögerung verschiedener Cellulosen | |
|---|---|
| Cellulose | Anquellzeit* |
| Walocel ® MW 20000 GB | 40 min |
| Cellulose **1A** | 50 min |
| Cellulose **1B** | 55 min |
| Natrosol® 250 H4BR | 20 min |
| Cellulose **2A** | 35 min |
| Cellulose **2B** | 45 min |

| | |
|---|---|
| * Anquellzeit: Zu 196,0 g VE-Wasser in einem 500 ml Becherglas wurden 4,0 g der Cellulose gegeben und mit einem Glasstab langsam verrührt. Es wird die Zeit bestimmt, bis zu der die Cellulosepulverteilchen in der Schwebe bleiben. | |

Die Viskosität dieser Lösungen wurde mit dem Haake VT 550 bei 23,0°C und 2,55s⁻¹ und 600 s⁻¹ bestimmt. Die Ergenisse sind in Tab. 2 wiedergegeben.

**Tabelle 2**

| Viskositäten von 2 %igen wäßrigen Lösungen verschiedener Cellulosen | | |
|---|---|---|
| Cellulose | Viskosität der 2 gew.%igen Lösung | |
| | 2,55 s⁻¹ | 600 s⁻¹ |
| Walocel® MW 20000 GB | 15000 mPas | 170 mPas |
| Cellulose 1A | 23500 mPas | 175 mPas |
| Cellulose 1B | 21000 mPas | 170 mPas |
| Natrosol®250 H4BR | 21000 mPas | 70 mPas |
| Cellulose 2A | 16500 mPas | 80 mPas |
| Cellulose 2B | 17500 mPas | 80 mPas |

### 3. Herstellung einer Innendispersionsfarbe mit den modifizierten Cellulosen

### 3.1. Innendispersionsfarben mit (modifizierten) niedrig veretherten Methylhydroxyethylcellulosen

Es wurden Innendispersionsfarben nach folgender Rezeptur hergestellt:
- 268,0 g: VE-Wasser
- 2,0 g: Calgon N Neu (10%ige Lösung in Wasser; Polyphosphat; BASF AG)
- 1,0 g: Nopco 8034 (Mineralölentschäumer; Henkel HGaA)
- 6,0 g: Cellulose
- 1,0 g: konz. Ammoniakwasser
- 5,0 g: Dispex GA 40 (Dispergiermittel; Allied Colloids)
- 5,0 g: Dowanol DPnB (Dipropylenglykol-n-butylether; Dow Chemical Corp.)
- 2,0 g: Preventol D6 (Konservierungsmittel; Bayer AG)
- 57,0 g: Kronos 2310 Titandioxid (Kronos Titan AG)
- 80,0 g: Socal P2 (synth. Calciumcarbonat; Solvay AG)
- 91,0 g: Finntalk M30SL (Talkum; Finntalk)
- 125,0 g: Omyalite 90 (Kreide; Omya GmbH)
- 297,0 g: Omyacarb 5 GU ( Calcit;Omya GmbH)

Diese Mischung wurde 15 min im Dissolver dispergiert. Dazu wurden dann 60,0 g Freihoff Styrolacrylatdispersion KD 524 U gegeben und 3 min mit dem Dissolver homogenisiert.

Dabei wurden folgende anwendungstechnische Daten erhalten (Tab. 3).

**Tabelle 3**

| anwendungstechnische Daten von Innenfarben mit verschiedenen Cellulosen | | | | |
|---|---|---|---|---|
| Cellulose | Viskosität bei 10,3 s⁻¹ [mPas] | Viskosität bei 600 s⁻¹ [mPas] | Spritzneigung* (Note) | Pigmentbenetzung** (Note) |
| Walocel® | | | | |
| MW 20000 GB | 12.000 | 120 mPas | 4 | 2 |
| Cellulose 1 A | 15.000 | 120 mPas | 2- | 1 |
| Cellulose 1 B | 14.500 | 120 mPas | 2- | 1 |

| | | | | |
|---|---|---|---|---|
| *: Spritzneigung: nach interner Prüfmethode (s.u.) | | | | |
| **: Pigmentbenetzung: Nach interner Prüfmethode (s.u.) | | | | |

### 3.2. Herstellung einer Innendispersionsfarbe mit Hydroxyethylcellulosen

Es wurden Innendispersionsfarben nach folgender Rezeptur hergestellt:
- 270,0 g: VE-Wasser
- 2,0 g: Calgon N Neu (10%ige Lösung in Wasser; Polyphosphat; BASF AG)
- 1,0 g: Nopco 8034 (Mineralölentschäumer; Henkel HGaA)
- 4,0 g: Cellulose
- 1,0 g: konz. Ammoniakwasser
- 5,0 g: Dispex GA 40 (Dispergiermittel; Allied Colloids)
- 5,0 g: Dowanol DPnB (Dipropylenglykol-n-butylether; Dow Chemical Corp.)
- 2,0 g: Preventol D6 (Konservierungsmittel; Bayer AG)
- 57,0 g: Kronos 2310 Titandioxid (Kronos Titan AG)
- 80,0 g: Socal P2 (synth. Calciumcarbonat; Solvay AG)
- 91,0 g: Finntalk M30SL (Talkum; Finntalk)
- 125,0 g: Omyalite 90 (Kreide; Omya GmbH)
- 297,0 g: Omyacarb 5 GU ( Calcit;Omya GmbH)

Diese Mischung wurde 15 min im Dissolver dispergiert. Dazu wurden dann 60,0 g Freihoff Styrolacrylatdispersion KD 524 U gegeben und 3 min mit dem Dissolver homogenisiert.

Dabei wurden folgende anwendungstechnische Daten erhalten (Tab.4).

**Tabelle 4**

| anwendungstechnische Daten von Innenfarben mit verschiedenen Cellulosen | | | | |
|---|---|---|---|---|
| Cellulose | Viskosität bei 10,3 s⁻¹ [mPas] | Viskosität bei 600 s⁻¹ [mPas] | Spritzneigung* (Note) | Pigmentbenetzung** (Note) |
| Natrosol® 250H4BR | 2440 | 200 | 4- | 2 |
| Cellulose 1 A | 2700 | 490 | 2- | 2 |
| Cellulose 1 B | 2900 | 490 | 2- | 2 |

| | | | | |
|---|---|---|---|---|
| *: Spritzneigung: nach interner Prüfmethode (s.u.) | | | | |
| **: Pigmentbenetzung: Nach interner Prüfmethode (s.u.) | | | | |

### 4. Prüfvorschrift zur Bestimmung der Spritzneigung von Dispersionsfarben

Geräte-Prüfmittel:
   Oberschalige Waage
   Stoppuhr
   Fellrolle 11cm breit, 2,5 cm Durchmesser mit Rollenhalter
   Lackwanne mit Abstreiffläche
   Eternitplatte 11cm x 20cm auf Lenettafolienzuschnitt 20cm x 30cm
   große Lenettafolie 30cm x 43cm
Reagenzien:
   keine weiteren Reagenzien nötig
Durchführung:
   Die saubere Fellrolle muß zunächst angefeuchtet und auf einem Handtuch trocken gerollt werden, damit alle Messungen unter gleichen Bedingungen stattfinden. In die Lackwanne werden 50-60g der zu prüfenden Dispersionsfarbe eingewogen. Anschließend wird die Waage auf 0 tariert und in die Fellrolle 30g +/- 0,1 g der Dispersionsfarbe eingewogen. Dabei ist darauf zu achten, daß die Farbe gut in die Rolle eingearbeitet wird. Die Eternitplatte mit der Lenettafolie wird in die Mitte der großen Lenettafolie gelegt, so daß nur auf dem oberen und unteren Teil der großen Lenettafolie Farbspritzer gelangen können. Nun wird die Rolle in gleichmäßigen Zügen 40 mal in 30 Sekunden längs auf der Eternittplatte hin und her gerollt. Dabei sollte die Rolle bis an die obere und untere Kante der Eternitplatte gerollt werden, ohne dabei von der Eternitplatte abzurutschen. Anschließend läßt man die Farbspritzer 5 min trocknen. Bei weiteren Prüfungen sollte die Eternitplatte vorher mit einem Kleenex-Tuch abgetupft werden, damit die auf der Eternitplatte verbleibende Farbe keinen Einfluß auf die anderen Messungen hat.
Auswertung:
   Die Auswertung erfolgt nach Menge und Größe der Farbspritzer. Dabei kann man sich nach folgenden Benotungen richten:
   1: nur vereinzelnte Spritzer
   2: wenig kleine Spritzer
   3: viele kleine Spritzer oder sehr wenig große Spritzer
   4: viele kleine Spritzer mit mehreren großen Spritzern
   5: viele kleine und große Spritzer
   6: sehr viele große Spritzer

### 5. Prüfvorschrift zur Bestimmung der Pigmentverteilung bei Dispersionsfarben

Prüfmethode: Interne Methode
Geräte-Prüfmittel:
   Oberschalige Waage
   Lenettafolie 297 mm x 210 mm
   100 µm Rakel
   180 ml Polybecher
Reagenzien:
   Luconyl-Violett 5894 (BASF)
   VE-Wasser
Durchführung:
   Zunächst wird eine Vorverdünnung des Luconyl-Violett 5894 1:10 in VE-Wasser zubereitet.
   Auf der oberschaligen Waage werden nun 99,5 g der zu prüfenden Dispersionsfarbe in den Polybecher eingewogen. Dieser setzt man dann 5 g des vorverdünnten Luconyl-Violett 5894 zu. Anschließend wird das Ganze von Hand gut verrührt. Das Luconyl-Violett muß gleichmäßig verteilt sein.
   Der Ansatz wird 10 min zum Ablüften stehen gelassen, nochmals vorsichtig verrührt und dann mit dem 100 µm Rakel längs auf der Lenettafolie aufgezogen.
   Nach 2 min. Standzeit kreist man seinen Zeigefinger vorsichtig 5 sek. lang auf
   einem Punkt des Farbaufzugs, ohne ihn dabei abzureiben (Rub-out). Die Lenettafolie darf danach nicht durch den Farbaufzug durchschimmern. Diesen Vorgang wiederholt man 3 mal alle 2 min. an einer neuen Stelle des Farbaufzugs. Anschließend läßt man den Film 1 Std. durchtrocknen.
Auswertung:
   Es wird der Farbkontrast zwischen der angeriebenen und der unberührten Fläche beurteilt.
   - Kriterien:: je größer der Farbkontrast ist, desto schlechter ist die Pigmentverteilung
   ( Pigmentaufschwemmung )
   je früher ein Farbkontrast auftritt, desto schlechter ist die Pigmentverteilung
   (schlechte Pigmentstabilität)

## Patentansprüche

1. Nichtionishe, wasserlösliche Cellulosen mit freien OH-Gruppen aus der Klasse der Methyl-, Hydroxyethyl-, Hydroxypropyl-, Carborymethyl-, Carboxyethyl-, Hydroxyethylmethyl-, Hydroxypropylmethyl- und Carboxyethylcellulosen umgesetzt mit polyfunktionellen Isocyanaten in einem Mengenverhältnis von 0,2 bis 10 Gew.%, bevorzugt 0,2 bis 5,0 Gew.% Isocyanat bezogen auf Cellulose.

2. Verfahren zur Herstellung der modifizierten Cellulosen gemäß Anspruch 1 durch Umsetzung von OH-funktionellen Cellulosen durch Reaktion mit polyfunktionellen Isocyanaten in organischen, inerten Lösemitteln.

3. Verfahren zur Herstellung der modifizierten Cellulosen nach Anspruch 2 unter Zuhilfenahme von Katalysatoren, die die Umsetzung von Isocynaten mit OH-Gruppen beschleunigen.

4. Verfahren zur Herstellung der modifizierten Cellulosen nach Anspruch 2 oder 3 bei erhöhter Reaktionstemperatur, bevorzugt von 40-100°C.

5. Verwendung von Cellulosen gemäß Anspruch 1 als verdickend wirkende Zusatzmittel in wässrigen Systemen, ausgewählt aus der Gruppe der wässrigen oder wasserverdünnbaren Industrielacke, Putz- und Anstrichsfarben, Druck- und Textilfarben, Pigmentdruckpasten, pharmazeutische oder kosmetische Formulierungen, Zubereitungen von Waschmitteln, Klebstoffen, Wachsen und Polituren.
